Europäisches Patentamt

European Patent Office

Office européen des brevets

(19)

(11) Publication number: **0 113 152**
**A2**

(12) **EUROPEAN PATENT APPLICATION**

(21) Application number: **83201855.0**

(22) Date of filing: **29.12.83**

(51) Int. Cl.³: **A 61 B 5/00**
**A 61 B 1/24, G 01 N 21/64**

(30) Priority: **03.01.83 US 455371**

(43) Date of publication of application:
**11.07.84 Bulletin 84/28**

(84) Designated Contracting States:
**DE FR GB IT NL SE**

(71) Applicant: **North American Philips Corporation**
**100 East 42nd Street 9th Floor**
**New York, N.Y. 10017(US)**

(72) Inventor: **Lafond, Ronald Joseph**
**c/o INT. OCTROOIBUREAU B.V. Prof. Holstlaan 6**
**NL-5656 AA Eindhoven(NL)**

(74) Representative: **Scheele, Edial François et al,**
**INTERNATIONAAL OCTROOIBUREAU B.V. Prof.**
**Holstlaan 6**
**NL-5656 AA Eindhoven(NL)**

(54) **Method and apparatus for examining anomalies on surfaces of objects.**

(57) Diseased areas of human teeth are identified by an orange-red fluorescence which is produced when the teeth are illuminated with visible light having a wavelength of less than approximately 434.5 nm. The teeth are viewed directly through an optical filter which blocks transmission at the illuminating wavelength. A peaking filter may be used to enhance the visibility of the fluorescence.

Fig. 4

Croydon Printing Company Ltd.

Method and apparatus for examining anomalies on surfaces of objects

## SCOPE OF THE INVENTION

The invention relates to methods and apparatus which make use of visible luminescence to produce images which delineate anomalies on surfaces of objects such as areas of human and animal teeth. Specifically, the invention identifies dental caries and calculus as fluorescent regions having distinct optical signatures.

## BACKGROUND OF THE INVENTION

United States Patent 4,290,433 to Alfano describes a method and apparatus for detecting the presence of caries in teeth using visible luminescence. Human teeth are excited with visible light at a first frequency (for example 410 ± 5 nm, thus in the blue region). The excitation causes the teeth to fluoresce. The wavelength of light emitted from caries is, in some regions of the spectrum, shifted in frequency with respect to light emitted from healthy regions of the tooth. Alfano scans the surface of an excited tooth with an optical fiber probe and utilizes electronic circuitry to determine the difference or ratio between light intensity in a first region of the spectrum which is unaffected by the presence of caries and in a second region of the spectrum wherein emision changes in the presence of caries. The output of the electronic circuitry may be displayed on a digital or analog meter or used to trigger an alarm light or buzzer to signal the presence of caries. Alfano also suggests that the teeth can be illuminated with monochromatic light and an image of fluorescent light, at a wavelength where the intensity changes in the presence of caries, may be formed and recorded on a suitable medium.

This method and apparatus do not appear to be clinically efficacious, using present technology, inasmuch as it is difficult to delineate diseased areas with the optical probe and recorded images are not usable in real-time, for example, during surgery.

Bjelkhagen, et. al. have described a technique for the early detection of enamel caries by the luminescence excited by visible laser light in the Swedish Dental Journal. Human teeth are illuminated by an argon laser at 488 nm in the blue-green region. The teeth are viewed and/or photographed through filters which transmit light at wavelengths longer than 540 nm. Caries appear as dark regions against the otherwise bright background of the tooth. The dark appearance of diseased regions makes them difficult to delineate in the presence of uneven illumination and during surgical procedures.

SUMMARY OF THE INVENTION

I have determined that anomalies  on the surface of objects such as dental caries and calculus on the surface of human teeth will fluoresce brightly and produce distinct visual signatures if the objects are excited with visible light within a critical spectral range. The anomalies  or diseased areas may be readily identified and differentiated from healthy tissue by direct viewing through suitable optical filters. The filters may be disposed in eyeglasses or goggles worn by an examiner, a dentist or technician in order to permit identification of, for example diseased tissue during dental surgery. In a preferred embodiment, the filters comprise optical glasses which block the exciting wavelength and have responses in bands which accentuate the differences between the optical signature of diseased and healthy tissue.

Some embodiments of the invention are described with reference to the attached drawing wherein:

Fig. 1 illustrates typical emission spectra which are derived from healthy and diseased dental tissue;

Fig. 2 illustrates the spectral response of the human eye;

Fig. 3 illustrates the spectral responses of a preferred blocking filter and a preferred peaking filter; and

Fig. 4 illustrates apparatus for practicing the invention.

## DESCRIPTION OF THE PREFERRED EMBODIMENT

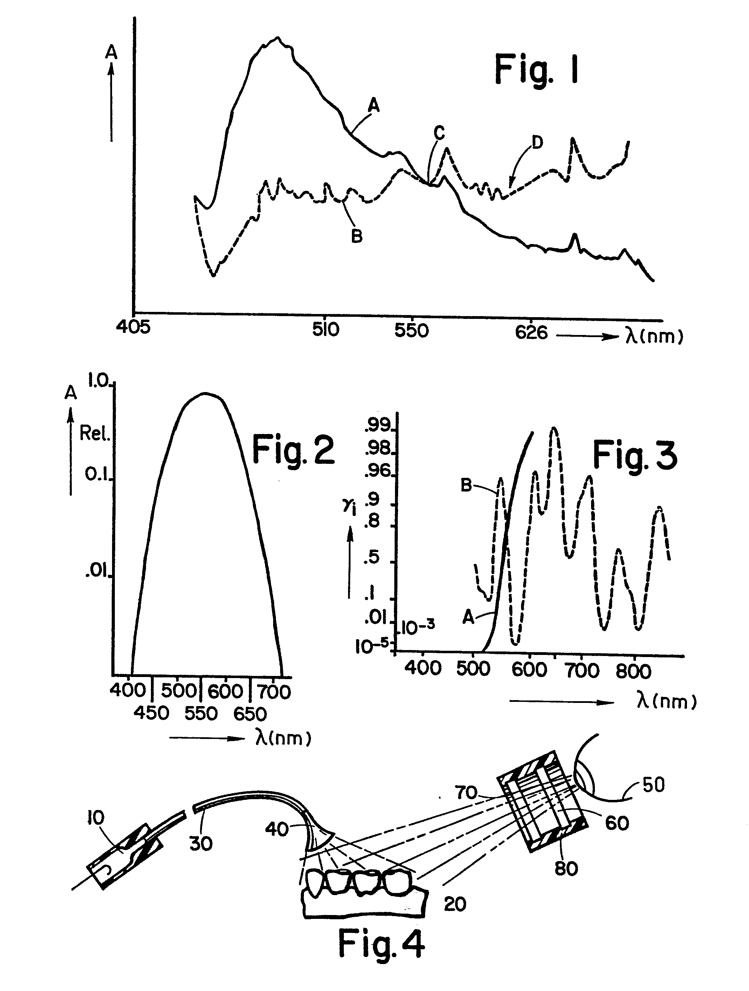

Curve A of Fig. 1 illustrates a typical emission spectrum from the healthy surface of an extracted tooth. Curve B of Fig. 1 illustrates a typical emission spectrum from dental caries in the same tooth. It may be noted that below a critical crossover wavelength C the intensity of light emitted from healthy tissue is greater than the intensity emitted from diseased tissue. Above the critical wavelength C the intensity of light emitted from the diseased tissue is greater than the intensity of light emitted from healthy regions of the tooth.

I have determined that the crossover wavelength C varies as a function of the wavelength of the light which is used to excite fluorescence in the tooth. Fig. 2 illustrates the spectral response of the human eye. If the wavelength of light used to excite the tooth is too great, the portion of the emitted spectrum D wherein increased luminescence distinguishes diseased tissue from healthy tissue will fall at wavelengths where the response of the human eye is too small to distinguish the resulting intensity differences. This is the case of the work of Bjelkhagen where excitation occurred at 488 nm. Caries then appear as dark regions and are particularly difficult to differentiate from shadows and amalgam fillings.

If, on the other hand, the excitation occurs within a critical range, as illustrated in Fig. 1, the crossover point lies near the peak spectral response of the human visual system and diseased regions of the tooth may be readily identified by a particularly bright orange-red fluorescence. The presence of a distinct colour signature which is associated with diseased areas permits easy identification in the presence of shadows or dark structures. It is particularly useful, for example, to identify diseased tissue within a tooth during surgical procedures. The presence of an orange-red glow allows the surgeon to excise only diseased portions of the tooth.

In a preferred embodiment of the invention, excitation is provided at 405 nm using a 150 watt mercury arc lamp. Excitation at the 434.5 mercury line produces marginal results using the present invention while excitation at 457 nm and above does not produce useful visual fluorescent identification of diseased regions of the tooth.

The excited teeth are viewed through an optical filter which blocks light at the excitation frequency. Typically, the filter is incorporated as a faceplate or goggles which are worn by the dentist or technician. An additional filter having transmission characteristics which accentuate the fluorescent signature of diseased tissue may be utilized in combination with the blocking filter.

In a preferred embodiment, with excitation at the 405 nm mercury line, the blocking filter comprises type OG550 sharp cut orange glass manufactured by Schott Optical Glass, Inc., Duryea, Pennsylvania having a transmission characteristic illustrated by curve A of Fig. 3 and the peaking filter comprises type BG36 Didymium multiband glass manufactured by Schott Optical Glass, Inc. and having a transmission characteristic illustrated by curve B of Fig. 3.

Fig. 4 schematically illustrates a preferred embodiment of the invention. Light from the mercury lamp 10 is directed onto the patient's teeth 20 through an optical fiber bundle 30 and probe 40. For surgical operations the probe may comprise a dental handpiece. An observer 50 views the illuminated teeth directly through the blocking filter 60 and peaking filter 70 which are contained in goggles 80.

# CLAIMS

1. A method for identifying diseased regions of objects such as teeth comprisng the steps of: illuminating the object with light having a wavelength less than or substantially equal to 434.5 nm, and directly observing orange-red fluorescence from anomalous regions of the illuminated object through an optical filter which blocks transmission of light at the exciting wavelength.

2. The method of Claim 1 wherein the step of directly viewing the fluorescence includes viewing the fluorescence simultaneously through an optical filter which blocks transmission at the excitation wavelength and through an optical filter which peaks transmission at the wavelength of the orange-red fluorescence.

3. The method of Claim 2 comprising the step of illuminating the object with monochromatic light.

4. The method of Claim 3 wherein the monochromatic light has a wavlength of 405 nm as dedicated to the examination of teeth.

5. The method of Claim 1 wherein the diseased regions comprise dental caries or calculus.

6. A method of dental surgery comprising identifying diseased areas according to the method of Claim 1 while surgically excising the identified diseased regions.

7. Apparatus for identifying anomalous regions of objects comprising: means for illuminating an object with light having a wavelength less than or substantially equal to 434.5 nm, and; an optical filter having a transmission characteristic which blocks light at the illuminating wavelength disposed between an observer and the object to permit direct viewing of orange-red fluorescence from anomalous regions of the object.

8. The apparatus of Claim 7 wherein the means for illuminating the object comprise a source of substantially

PHA 21.148       6       0113152

monochromatic light.

9.      The apparatus of Claim 7 wherein the light has a wavelength of 405 nm and the apparatus is dedicated to the examination of human teeth.

10.      The apparatus of Claim 7 or 9 wherein the means for illuminating the tooth comprises a mercury arc lamp; an optical fiber bundle coupled to the arc lamp to transmit light therefrom; dental probe means adapted to project light from the optical fiber onto the tooth and means for carrying optical filters adapted to the light to be examined.

Fig. 1

Fig. 2

Fig. 3

Fig. 4